(19) Europäisches Patentamt

European Patent Office

Office européen des brevets

(11) EP 0 754 028 B1

(12) EUROPÄISCHE PATENTSCHRIFT

(45) Veröffentlichungstag und Bekanntmachung des Hinweises auf die Patenterteilung:
06.09.2000 Patentblatt 2000/36

(21) Anmeldenummer: 95914337.1

(22) Anmeldetag: 31.03.1995

(51) Int Cl.7: A61K 7/32

(86) Internationale Anmeldenummer:
PCT/EP95/01213

(87) Internationale Veröffentlichungsnummer:
WO 95/26708 (12.10.1995 Gazette 1995/43)

(54) **NEUE DESODORIERENDE UND ANTIMIKROBIELLE ZUSAMMENSETZUNGEN ZUR VERWENDUNG IN KOSMETISCHEN ODER TOPISCHEN ZUBEREITUNGEN**

NOVEL DEODORISING AND ANTI-MICROBIAL COMPOSITIONS FOR USE IN COSMETIC OR TOPICAL PREPARATIONS

NOUVELLES COMPOSITIONS DESODORISANTES ET ANTIMICROBIENNES DESTINEES A ETRE UTILISEES DANS DES PREPARATIONS COSMETIQUES OU TOPIQUES

(84) Benannte Vertragsstaaten:
AT BE CH DE ES FR GB IT LI NL

(30) Priorität: 05.04.1994 DE 4411664

(43) Veröffentlichungstag der Anmeldung:
22.01.1997 Patentblatt 1997/04

(73) Patentinhaber: Beiersdorf Aktiengesellschaft
20245 Hamburg (DE)

(72) Erfinder:
• HOPPE, Udo
D-22397 Hamburg (DE)
• LIEBL, Martina
D-21129 Hamburg (DE)
• SAUERMANN, Gerhard
D-24649 Wiemersdorf (DE)
• TRAUPE, Bernd
D-22457 Hamburg (DE)
• WOLF, Florian
D-20251 Hamburg (DE)

(56) Entgegenhaltungen:
EP-A- 0 297 310          DE-A- 4 319 546

## Beschreibung

Die Erfindung betrifft desodorierende und antimikrobielle Zusammensetzungen zur Verwendung in kosmetischen oder topischen Zubereitungen, insbesondere desodorierenden kosmetischen Mitteln.

Desodorierende kosmetische Mittel werden insbesondere zur Unterdrückung von unangenehmem Körpergeruch eingesetzt, welcher durch die Einwirkung bestimmter Hautbakterien unter dem Einfluß von Wärme und Feuchtigkeit auf den zunächst weitgehend geruchlosen apokrinen Schweiß infolge der Bildung von stark riechenden Zersetzungsprodukten entsteht.

Zur Zeit sind neben Molekülen, die Gerüche absorbieren, zwei Hauptklassen von Produkten zur Bekämpfung schlechter Gerüche aufgrund des Schwitzens bekannt.

Einmal sind Antitranspirationsmittel auf der Grundlage von Produkten bekannt, die die Schweißbildung unterbinden oder stark hemmen, wie Adstringentien auf der Grundlage von Aluminiumsalzen und insbesondere auf der Grundlage von Aluminiumhydroxychlorid. Mit diesen Mitteln kann man die Bildung schlechter Gerüche unterbinden, indem man ihre unmittelbare Ursache, nämlich die Entwicklung von Schweiß durch die Epidermis unterdrückt (vgl. DE-OS 21 37 926). Im Gegensatz zu diesen Antitranspirantien handelt es sich bei kosmetischen Mitteln mit desodorierender Wirkung um eine Klasse von Mitteln, die zwar nicht oder nur schwach auf das Schweißvolumen einwirken, die jedoch aufgrund ihrer bakteriziden Wirkung die Bakterien, die zur Zersetzung des Schweißes führen, zerstören. Hierzu zählen Mittel mit einem Gehalt an antimikrobiellen Stoffen. Unter den Verbindungen mit derartigen Eigenschaften wurden z.B. Phenolderivate mit und ohne Halogensubstituenten, organische Quecksilberverbindungen, quartäre Ammoniumverbindungen, wie Cequartyl$^{(R)}$ oder bestimmte Ionenaustauscher oder Metallchelate von 1,3-Diketonen sowie desinfizierend wirkende Abkömmlinge von Aminosäuren bekannt.

Ferner wurden Phenylhydroxyalkylether, insbesondere die unter der Bezeichnung Phenoxyethanol bekannte Verbindung aufgrund ihrer bakteriziden und fungiziden Wirkungen auf eine Anzahl von Mikroorganismen als Konservierungsmittel eingesetzt. Phenoxyethanol ist vor allem in saurem und neutralem, aber auch im alkalischen Milieu wirksam und völlig ungiftig. Es gibt bereits in niedrigen Konzentrationen ausreichend Schutz. Aufgrund seines neutralen Geschmacks fand es schnell Eingang in die pharmazeutische und kosmetische Industrie. Seine Wirkung richtet sich allerdings hauptsächlich gegen gram-negative Bakterien.

In chemischer Sicht verhält sich Phenoxyethanol bei seiner Verwendung weitgehend indifferent. Es stellt eine farblose, leicht viskose Flüssigkeit von schwachem, angenehmen Geruch und einem zusammenziehenden Geschmack dar, ist mischbar mit Aceton, Ethylalkohol und Glycerin, löslich in Wasser (1 : 45) und Fetten, z.B. Oliven- und Erdnußöl (1 : 50).

Die Löslichkeit von Phenoxyethanol in Wasser ist jedoch gering und reicht für manche Konservierungszwecke nicht aus.

Phenoxyethanol, das in der Literatur hinreichend beschrieben ist, wurde in der Natur nachgewiesen in tropischen Früchten in Cichorium endivia sowie in grünem Tee (Camellia sinesis). Es hat einen milden, rosenähnlichen Duft und wird für Parfümkompositionen auch als Fixatur eingesetzt.

Aus der GB-PS 11 55 789 ist es auch bekannt, bestimmte Phenylether als antibakterielle Mittel in Reinigungszusammensetzungen für die Haut einzusetzen. Außerdem finden substituierte Phenylether als antibakterielle Mittel Verwendung (vgl. DE Offenlegungsschrift 16 42 057).

In Weiterentwicklung des zuvor aufgezeigten Prinzips wurde daher versucht, zusätzlich die antimikrobiellen Eigenschaften bestimmter Riechstoffe, etherischer öle oder anderer Parfümbestandteile zu nutzen und diese als antimikrobielle und desodorierende Wirkstoffe in desodorierenden Parfümkompositionen einzusetzen. Als eine derartige antimikrobiell wirksame Substanz, die das Wachstum geruchsbildender Bakterien auf der Haut stark hemmt, ohne die gesamte Bakterienflora der Haut stark zu verändern, beschreiben die DE-OS 27 28 9Z1 und die DE-OS 33 15 058 den Naturstoff Farnesol (z.B. 2-trans, 6-trans-3,7,11-Trimethyldodeca-2,6,10-trien-1-ol) und seine 3 synthetischen Isomere. Nachteilig ist dabei jedoch, daß diese Verbindungen bei Verwendung als desodorierender, antimikrobieller Wirkstoff in wesentlich höheren Konzentrationen eingesetzt werden müssen als in üblichen Parfümzusammensetzungen, um die erwünschte desodorierende Wirkung zu erzielen.

So sind beispielsweise für eine völlige Wachstumshemmung bei den grampositiven Bakterien Staphylococcus aureus und Staphylococcus epidermidis sowie für eine weitgehende Hemmung gegenüber Corynebacterium spec. eine Konzentration von mindestens 0,3 Gew.-% Farnesol, bezogen auf die kosmetische Zusammensetzung erforderlich. In Riechstoffkompositionen und in desodorierend wirkenden Produkten beträgt der Gehalt an Farnesol 0,2 bis 0,5 %.

Das Farnesol (3,7,11-Trimethyldodeca-2,6,10-trienol-1) ist ein acyclischer primärer Sesquiterpenalkohol, dessen natürliches Vorkommen in der Literatur hinreichend dokumentiert wurde. So findet man es im Lemongrasöl, Palmarosaöl, Citronellöl, Tuberosenblütenöl, Sandelholzöl, Lindenblütenöl und in vielen anderen Naturstoffen.

Es ist eine farblose Flüssigkeit mit typischem Geruch, klar löslich in 3 Teilen Ethylalkohol (70%).

Als Hilfsstoff zur Konservierung in kosmetischen Mitteln sind auch schon Glycerinmonoester kurzkettiger und mittelkettiger Fettsäuren bekannt.

Außerdem hat als ein für kosmetische Mittel geeignetes Germicid das Glycerinmonolaurat, bekannt unter der Handelsbezeichnung Lauricidin(R) (Dragoco) zu gelten. Es ist dispergierbar in Wasser, löslich in Alkohol, Fetten und Paraffinöl, mischbar mit Aceton.

Solche Glycerinmonoester und Glycerinmonolaurat sind in der Natur zumindest als Stoffwechselprodukte bei der Verdauung von Speisefetten nachgewiesen. Verschiedene Monoglyceride sind daher als Zusatzstoffe in der Lebensmittelindustrie üblich. Glycerinmonolaurat und Glycerinmonocaprylat finden als pharmazeutische Salbengrundlage, als Co-Emulgatoren für Emulsionen und als konsistenzgebende Komponenten für verschiedenste kosmetische Mittel wie Shampoo, Badezusätze, Cremes oder Lotionen Verwendung.

Diese vorstehend aufgeführten zwei Klassen von Mitteln sind jedoch nicht vollständig befriedigend, weil einerseits die adstringierenden Mittel oder Antitranspirationsmittel das natürliche Phänomen der Schweißbildung unterbinden und darüber hinaus eine ungünstige Wirkung auf die Epidermis ausüben und andererseits ein Teil der bakteriziden Mittel den Nachteil aufweisen, daß sie vollständig die Mikrobenflora der Haut zerstören und demzufolge das biologische Gleichgewicht der Epidermis empfindlich stören.

Hinzu kommt, daß die Mehrzahl dieser Mittel einen leicht phenolischen Geruch besitzt. Aus diesem Grunde gilt weiterhin das Bestreben, sehr gut desodorierende, geruchsneutrale und von Nebenwirkungen freie kosmetische Mittel herzustellen.

Zwar werden in neuerer Zeit Deodorantien bekannt, welche auf die angeführten traditionellen Wirkstoffe verzichten. Beispielsweise versucht man das Deo-Problem ausschließlich über das Parfüm zu lösen. Dabei sollen die Körpergeruchskomponenten gewissermaßen als Duftkomplex vom Parfüm so neutralisiert werden, daß der nachteilige Körpergeruch einige Zeit übertönt wird.

Die Wirkung dieser desodorierenden kosmetischen Mittel ist jedoch, was Wirkstärke (Geruchsmaskierung) und Wirkungsdauer angeht, für die Bedürfnisse der Praxis nicht ausreichend.

Weiterhin nutzt man die antibakteriellen Eigenschaften bestimmter Riechstoffe, ätherischer Öle oder anderer Parfümbestandteile einzeln oder in Mischung, indem man desodorierende Parfümkompositionen als solche konfektioniert. Derartige Produkte wirken sowohl über den Duft als auch über die antibakterielle Wirkung über einen längeren Zeitraum desodorierend.

Letztlich ist noch eine Gruppe von Substanzen zu nennen, welche über eine Enzymhemmung verhindern, daß unangenehm riechende Zersetzungsprodukte aus den Schweißinhaltsstoffen, Hornschichtresten und Hautoberflächenfett entstehen.

Aber selbst wenn bei Einsatz von Deodorantien die Gefahr von Hautreizung nicht in dem Maße wie bei Verwendung von Antitranspirantien hervorgerufen wird, so treten auch bei laufender Benutzung von Deodorantien mitunter Unverträglichkeiten, Lichtsensibilisierungen und toxische Nebenwirkungen verschiedener Intensität auf.

Häufiger Nachteil derartiger desodorierender Wirkstoffe ist, daß nicht nur die für den Körpergeruch verantwortlichen Bakterien am Wachstum gehindert oder abgetötet werden, sondern darüber hinaus auch andere Bakterien der bakteriellen Hautflora vernichtet werden. Derartige desodorierende Wirkstoffe sind also in unerwünschter Weise wesentlich stärker wirksam, als zur Vermeidung von Körpergeruch notwendig wäre.

Eine desodorierend wirkende Kombination der vorstehend beschriebenen Wirkstoffe Phenoxyethanol, Farnesol und Glycerinmonolaurat ist bereits aus der europäischen Patentanmeldung EP-A-297 310 bekannt. Sie hat sich aber nicht immer in allen Aspekten als so voll zufriedenstellend erwiesen, daß keine Verbesserungen denkbar wären.

An ein voll zufriedenstellendes Deo-Mittel werden folgende Voraussetzungen geknüpft:

1) Schonung der natürlichen Biologie der Haut
2) Duftneutralität
3) hohe Wirksamkeit nur in Bezug auf Desodorierung, d.h. nur Vermeidung und/oder Beseitigung von Körpergeruch
4) Vermeidung der Bildung von resistenten Bakterienstämmen
5) Unschädlichkeit bei Überdosierung oder sonstiger nicht bestimmungsgemäßer Anwendung
6) Gute kosmetische Anwendung und Performance
7) Leichte Handhabung (z.B. als Flüssigkeit) und universelle Verwendbarkeit in verschiedensten kosmetischen und externen Zubereitungen
8) Ausgezeichnete Haut- und Schleimhautverträglichkeit
9) Einsatz umweltfreundlicher Stoffe
10) Rückgriff auf natürliche Systeme oder in der Natur vorkommender Stoffe mit Status (GRAS, RFM etc.)
11) Pufferkapazität

Aufgabe der Erfindung war es daher, eine neue, hochwirksame desodorierende oder antimikrobielle Zusammensetzung auf der Basis von in der Natur vorkommenden oder naturnahen Einsatzstoffen, wie z.B. etherischen Ölen oder Duftstoffen, zu schaffen, die bei möglichst weitgehender Schonung der natürlichen Biologie der Haut wirksam desodoriert, universell in verschiedensten desodorierenden kosmetischen Mitteln eingesetzt werden kann und dabei z.B. geringere Einsatzmengen erfordern kann, als der bisher bekannte Stand der Technik vorsieht.

Es wurde gefunden und darin liegt die Lösung dieser Aufgabe, daß eine Zusammensetzung aus einem oder mehreren 3,7,11-Trimethyl-2,6,10-dodecatrien-1-olen, einem Phenylhydroxyalkylether mit einem, zwei oder drei C-Atomen im Alkylrest und mindestens einem Glycerinmonoester einer kurzkettigen oder mittelkettigen Fettsäure die obengenannten Anforderungen erfüllt, wobei diese Zusammensetzung gegebenenfalls noch zusätzlich Glycerinmonolaurat enthalten kann.

Gegenstand der Erfindung sind daher desodorierende oder antimikrobielle Zusammensetzungen, insbesondere zur Verwendung in kosmetischen oder topischen Zubereitungen, dadurch gekennzeichnet, daß sie

a) mindestens ein 3,7,11-Trimethyl-2,6,10-dodecatrien-1-ol,
b) mindestens einen Phenylhydroxyalkylether mit einem, zwei oder drei Kohlenstoffatomen im Alkylrest,
c) mindestens einem Glycerylmonoester einer Fettsäure mit 3-11 Kohlenstoffatomen und
d) gegebenenfalls Glycerinmonolaurat enthalten.

Die erfindungsgemäßen Zusammensetzungen mit den Komponenten a), b) und c) enthalten beispielsweise, jeweils bezogen auf das Gesamtgewicht der Zusammensetzungen,

a) 10 - 50, vorzugsweise 15 - 40 Gew.-% eines oder mehrerer 3,7,11-Trimethyl-2,6,10-dodecatrien-1-ole,

b) 5 - 45, vorzugsweise 10 - 35 Gew.-%, mindestens eines Phenylhydroxyalkylethers mit maximal 3 C-Atomen im Alkylrest,

c) 10 - 80, vorzugsweise 25 - 75 Gew.-% mindestens eines Glycerinmonoesters einer kurzkettigen oder mittelkettigen Fettsäure,

wobei die Mengen so auszuwählen sind, daß die Summe von a), b) und c) 100 Gew.-% ergibt.

In Zusammensetzungen, die neben den drei erfindungsgemäßen Komponenten a), b) und c) auch noch die vierte erfindungsgemäße Komponente d), Glycerinmonolaurat, enthalten, werden die Gewichtsanteile der Komponenten a), b) und c) variiert, wobei die Gewichtsmengen so auszuwählen sind, daß die Summe von a), b), c) und d) 100 Gew.-% ergibt.

Vorzugsweise beträgt der Gewichtsanteil von Glycerinmonolaurat 1 bis 20 Gew.-%, insbesondere 5 bis 15 Gew.-%, jeweils bezogen auf das Gesamtgewicht der vier Komponenten.

Erfindungsgemäße Zusammensetzungen mit den Komponenten a), b), c) und d) enthalten beispielsweise, jeweils bezogen auf das Gesamtgewicht der Zusammensetzungen,

a) 10 - 45, vorzugsweise 15 - 35 Gew.-% eines oder mehrerer 3,7,11-Trimethyl-2,6,10-dodecatrien-1-ole,

b) 5 - 40, vorzugsweise 10 - 30 Gew.-%, mindestens eines Phenylhydroxyalkylethers mit maximal 3 C-Atomen im Alkylrest,

c) 10 - 70, vorzugsweise 25 - 65 Gew.-% mindestens eines Glycerinmonoesters einer Fettsäure mit 3-11 Kohlenstoffatomen,

d) 1 - 20, vorzugsweise 5 - 15 Gew.-% Glycerinmonolaurat,

wobei die Mengen so auszuwählen sind, daß die Summe von a), b), c) und d) 100 Gew.-% ergibt.

Die verschiedenen 3,7,11-Trimethyl-2,6,10-dodecatrien-1-ole, bevorzugt der Naturstoff Farnesol und seine geometrischen Isomere, können dabei einzeln oder in Form jeder beliebigen Mischung eingesetzt werden.

Bei den in den erfindungsgemäßen Zusammensetzungen eingesetzten Phenylhydroxyalkylethern mit 1 bis 3 C-Atomen im Alkylrest handelt es sich bevorzugt um solche, bei denen sich die Hydroxygruppe am Alkylrest in der Endposition befindet. Es kann ein Ether oder ein Gemisch mehrerer Ether verwendet werden. Besonders bevorzugt ist der Einsatz von Phenoxyethanolen (Ethylenglykolmonophenylether), z.B. 2-Phenoxyethanol. Es wird z.B. in Mengen von 5 bis 45 Gew.-%, vorzugsweise 10 bis 35 Gew.-%, vorzugsweise einzeln aber auch in Mischung mit diesen anderen Ethern in der erfindungsgemäßen desodorierenden antimikrobiellen Zusammensetzung verwendet.

Geeignete Glycerinmonoester einer kurzkettigen oder mittelkettigen Fettsäure enthalten Fettsäuren mit 3 - 11 Kohlenstoffatomen, besonders bevorzugt 6 - 10 Kohlenstoffatomen, insbesondee 6 - 8 Kohlenstoffatomen. Die Fettsäuren sind vorzugsweise gesättigt. Dei Alkyle können geradkettig oder verzweigt sein, bevorzugt wird geradkettiges Alkyl. Vorzugsweise ist eine endständige OH-Gruppe des Glycerins verestert. Es kann ein Glycerinmonoester oder ein Gemisch mehrerer Ester verwendet werden.

Besonders bevorzugt wird Glycerinmonocaprylat. Es ist unter der Handelsbezeichnung Monocaprylin (Fa. Straetmanns) erhältlich . Es ist vorzugsweise an der endständigen OH-Gruppe verestert.

Glycerinmonolaurat ist bekannt und unter der Handelsbezeichnung Lauricidin (Fa. Dragoco) erhältlich. Vorzugsweise ist die endständige OH-Gruppe verestert.

Zwar waren auch für Ethylenglykolmonophenylether (U.S. Patent 2,451,149) und Glycerinmonolaurat schon gewisse antimikrobielle Eigenschaften bekannt, die erfindungsgemäßen Zusammensetzungen erwiesen sich aber in überraschender und nicht vorherzusehender Weise als signifikant wirksamer, als dies für die Summe der Einzelkomponenten zu erwarten war.

Bei mikrobiologischen Untersuchungen wurde für Keime, die für die Deowirkung relevant sind, eine synergistische Wirkung der erfindungsgemäßen Zusammensetzungen in der Form nachgewiesen, daß eine wirksame Menge der erfindungsgemäßen Zusammensetzungen deutlich mikrobizider wirkte, als die Summe der Wirksamkeiten der einzelnen Komponenten erwarten ließ. Die synergistische Wirkung wurde anhand mikrobiologischer Wirksamkeitstets bestätigt.

Daher sind die erfindungsgemäßen desodorierenden und antimikrobiellen Zusammensetzungen bei Verwendung in topischen oder kosmetischen Zubereitungen verglichen mit den Einzelkomponenten auch z.B. bei geringeren Einsatzmengen ausreichend wirksam oder bei gleichen Mengen stärker wirksam oder längere Zeit wirksam.

Mit den erfindungsgemäßen Zusammensetzungen erhaltene Deomittel können zur Behandlung der Achseln, der Füße, der Körperoberfläche oder der Haare oder des Genitalbereichs verwendet werden. Die Anwendung erfolgt in bekannter Weise duch einmaliges oder mehrmaliges Auftragen täglich.

Ein weiterer Vorteil der erfindungsgemäßen Zusammensetzungen besteht darin, daß sie problemlos in den verschiedenen Typen von Rezepturen für desodorierende kosmetische Mittel wie z.B. Roll-on, Stift, Puder, Lotion, Spray oder Puderspray, Deoseife oder Lösung eingesetzt werden können.

Die direkte Einarbeitung der erfindungsgemäßen synergistischen Zusammensetzungen in externe Zubereitungen und topische und kosmetische Mittel besitzt den Vorteil, daß eine homogene Verteilung der Komponenten gewährleistet ist und somit der zeitaufwendige Einsatz der Einzelkomponenten ausgeschaltet wird.

Eine bevorzugte Ausführungsform der Erfindung stellen daher desodorierende kosmetische Mittel dar, die neben üblichen Bestandteilen als desodorierenden Wirkstoff eine wirksame Menge der erfindungsgemäßen Zusammensetzungen enthalten. Als besonders vorteilhaft haben sich bei dieser Ausführungsform der Erfindung desodorierende kosmetische Mittel gezeigt, die z.B. einen Gehalt von 0,01 bis 10 Gew.-%, vorzugsweise einen Gehalt von 0,05 bis 5,00 Gew.-%, insbesondere 0,1 bis 0,9 Gew.-%, bezogen auf die Gesamtmenge des kosmetischen Mittels, der erfindungsgemäßen desodorierenden und antimikrobiellen Zusammensetzungen aufweisen.

Chemilumineszenzmessungen auf der Haut legen dabei nahe, daß die gute desodorierende Wirkung der erfindungsgemäßen Zusammensetzungen neben der antimikrobiellen Wirkung auch auf oxidative Reaktionen zurückzuführen ist, die durch die erfindungsgemäße Zusammensetzung ausgelöst werden.

Mit den erfindungsgemäßen Zusammensetzungen und Mitteln werden alle gestellten Aufgaben gelöst.

Die erfindungsgemäßen kosmetischen Desodorantien mit den erfindungsgemäßen Zusammensetzungen können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, als Deo-Stifte (Deo-Sticks) und in Form von aus normalen Flaschen und Behältern auftragbaren W/O-oder O/W-Emulsionen, z.B. Crèmes oder Lotionen. Weiterhin können die kosmetischen Desodorantien vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Als übliche kosmetische Trägerstoffe zur Herstellung der erfindungsgemäßen desodorierenden Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen kosmetischen Desodorantien, welche vorteilhaft als flüssige Zubereitungen mittels einer Roll-on-Vorrichtung auf die gewünschten Hautbereiche aufgetragen werden sollen, und die in den Zubereitungen in geringer Menge, z.B. 2 bis 5 Gewichts.-%, bezogen auf die Gesamt-Zusammensetzung, verwendet werden können, haben sich nichtionogene Typen, wie Polyoxyethylen-Fettalkoholether, z.B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den desodorierenden kosmetischen Zubereitungen gemäß der Erfindung, deren pH-Wert vorzugsweise z.B. durch übliche Puffergemische auf 4,0 bis 9,0 insbesondere 5,0 bis 6,5, eingestellt wird, Parfüm, Farbstoffe, Antioxidantien (z.B. $\alpha$-Tocopherol und seine Derivate oder Butylhydroxytoluol (BHT = 2,6-Di-Tert.-butyl-4-methylphenol) in Mengen von 0,01 bis 0,03 %, bezogen auf die Gesamtzusammensetzung), Suspendiermittel, Puffergemische oder andere übliche kosmetische Grundstoffe beigemischt werden.

Vorteilhaft wird der pH-Wert der erfindungsgemäßen Formulierungen im schwach sauren bis schwach alkalischen Bereich eingestellt, bevorzugt von 4,0 - 9,0 , besonders bevorzugt von 5,0 - 6,5.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Zur Parfümierung sind gegebenenfalls auch solche Substanzen und Parfümöle geeignet, die stabil sind, die Haut nicht reizen und bereits als solche antibakterielle oder bakteriostatische Eigenschaften besitzen.

Die Herstellung der kosmetischen Zubereitungen erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von kosmetischen Formulierungen zu beachten, die dem Fachmann geläufig sind.

Sollen die erfindungsgemäßen Ester in Pudersprays eingearbeitet werden, so können die Suspensionsgrundlagen dafür vorteilhaft gewählt werden aus der Gruppe

Kieselsäuregele (z.B. solche die unter dem Handelsnamen Aerosil$^R$ erhältlich sind), Kieselgur, Talkum, modifizierte Stärke, Titandioxid, Seidenpulver, Nylonpulver, Polyethylenpulver und verwandten Stoffen.

Eine weitere vorteilhafte Ausführungsform der Erfindung stellt die Verwendung der erfindungsgemäßen desodorierenden und antimikrobiellen Zusammensetzungen als antimikrobielle Wirkstoffe zur Stabilisierung topischer oder kosmetischer Zubereitungen gegen die Zersetzung durch Mikroorganismen dar.

Es können die oben angegebenen Mengen verwendet werden. Besonders bevorzugt werden Mengen von 0,3 - 0,75 Gew.-% bezogen auf das Gesamtgewicht.

Im Rahmen der vorliegenden Anmeldung sind, soweit nicht anders angegeben, Mengen und Prozentangaben auf das Gewicht und die Gesamtzusammensetzung der jeweiligen Zubereitungen bezogen.

Die nachfolgenden Beispiele sollen die Erfindung erläutern, ohne daß aber beabsichtigt ist, die Erfindung auf diese Beispiele zu beschränken.

Beispiel 1

In einem Lösekessel mit Rühreinrichtung wurden die folgenden Mischungen A - F zusammengegeben und bei Raumtemperatur gerührt, bis eine homogene Lösung entstanden war:

| Zusammensetzung: | A | B | C | D | E | F |
|---|---|---|---|---|---|---|
| Komponente I | 34 | 34 | 10 | 30 | 25 | 8 |
| II | 13 | 25 | 75 | 13 | 25 | 75 |
| III | 0 | 0 | 0 | 10 | 7 | 7 |
| IV | 53 | 41 | 15 | 47 | 43 | 10 |
| Komponente I: 3,7,11-Trimethyl-2,6,10-dodecatrien-1-ol (Isomerengemisch aus 4 Isomeren, Farnesol Nr. 2/027040 der Fa. Dragoco, Holzminden) II: Glycerinmonocaprylat-(1) (Monocaprylin) III: Glycerinmonolaurat-(1) (Lauricidin) IV: 2-Phenoxyethanol | | | | | | |

Die antimikrobielle Wirksamkeit der obengenannten erfindungsgemäßen Zusammensetzungen wurde im Vergleich zu den Einzelkomponenten mit Hilfe von Absterbekinetiken untersucht. Die Substanzen wurden als Lösungen oder Dispersionen in Wasser in Verdünnungsreihen eingesetzt.

Die in den Beispielen 2 bis 10 aufgeführten desodorierenden kosmetischen Mittel wurden nach den für die jeweiligen Mittel üblichen, dem Fachmann bekannten Verfahren hergestellt. Dabei bedeutet die Abkürzung GT Gewichtsteile, die Abkürzung EO steht für Ethylenoxid-Einheiten.

Als erfindungsgemäße Zusammensetzung A, B, C, D, E oder F wird in den folgenden Beispielen immer jeweils eine dieser in Beispiel 1 genannten Zusammensetzungen (A - F) in der angegebenen Gewichtsmenge eingesetzt, so daß nacheinander alle Zusammensetzungen (einzeln) verwendet werden.

Beispiel 2

| Deodorant Roll-on: | |
|---|---|
| Methylcellulose (Viskontran$^R$ HEC 30 000) | 0,80 GT |
| Wasser | 52,00 GT |
| Ethoxyliertes Glycerinmonococoat 7EO (Cetiol$^R$ HE) | 1,00 GT |
| Hydriertes Rizinusöl 40 EO (Cremophor$^R$ RH40) | 2,50 GT |
| Ethanol | 39,20 GT |
| 1,2-Propylenglykol | 3,00 GT |
| Parfüm | 1,00 GT |
| Erfindungsgem. Zusammensetzung A, B, C, D, E oder F nach Beispiel 1 | 0,15 GT |
| Farblösung 0,025%ig | 0,35 GT |

Beispiel 3

| Desodorierender Stift: | |
|---|---|
| 1,2-Propylenglykol | 46,00 GT |
| Stearinsäure | 7,00 GT |
| Ethylalkohol | 35,10 GT |
| Wasser | 10,00 GT |
| NaOH-Plätzchen | 1,20 GT |
| Parfüm | 0,50 GT |
| Erfindungsgem. Zusammensetzung A, B, C, D, E oder F nach Beispiel 1 | 0,20 GT |

Beispiel 4

| Desodorierende lotion (dickflüssig): | |
|---|---|
| Polyethylenglykol(20)oleylether (Cremophor$^R$O) | 2,00 GT |
| Cetylstearylalkohol | 3,00 GT |
| Paraffinöl | 5,00 GT |
| 1,2-Propylenglykol | 3,00 GT |
| Polyvinylpyrrolidon (Luviskol$^R$K30) | 0,50 GT |
| Erfindungsgem. Zusammensetzung A, B, C, D, E, oder F nach Beispiel 1 | 0,15 GT |
| Wasser | 89,90 GT |
| Parfüm | 0,45 GT |

Beispiel 5

| Desodorierende Lotion (dünnflüssig): | |
|---|---|
| Ethoxylierter Fettalkohol 6 EO (Cremophor[R]A) | 1,00 GT |
| Polyethylenglykol(20)oleylether (Cremophor[R]O) | 1,00 GT |
| Glycerinmonostearat | 2,00 GT |
| Cetylalkohol | 1,00 GT |
| Isopropylmyristat | 2,00 GT |
| Glycerin | 1,00 GT |
| Polyvinylpyrrolidon (Luviskol[R]K30) | 0,50 GT |
| Erfindungsgem. Zusammensetzung A, B, C, D, E oder F nach Beispiel 1 | 0,15 GT |
| Wasser | 90,90 GT |
| Parfüm | 0,45 GT |

Beispiel 6

| Desodorierendes Pumpspray (nicht Aerosol) | |
|---|---|
| Ethanol | 61,50 GT |
| Ethoxyliertes Glycerinmonococoat7EO (Cetiol[R]HE) | 1,50 GT |
| Erfindungsgem. Zusammensetzung A, B, C, D, E oder F nach Beispiel 1 | 0,40 GT |
| Citronensäure | 0,02 GT |
| Wasser | 36,18 GT |

Beispiel 7

| Desodorierendes Körperspray (Aerosol) | |
|---|---|
| Ethanol | 21,35 GT |
| 1,2-Propylenglykol | 3,00 GT |
| Octyldodecanol (Eutanol[R]G) | 0,04 GT |
| Parfüm | 0,50 GT |
| Erfindungsgem. Zusammensetzung A, B, C, D, E oder F nach Beispiel 1 | 0,10 GT |
| Isopropylmyristat | 0,01 GT |
| Treibgas | 75,00 GT |

Beispiel 8

| Desodorierende Intim-Waschlösung | |
|---|---|
| Cocoamidopropyl-Betain 30%ig (Tego-Betain[R]L7) | 10,00 GT |
| Ethoxyliertes Glycerinmonolaurat 22EO (Tagat[R]L2) | 2,00 GT |

(fortgesetzt)

| Desodorierende Intim-Waschlösung | |
|---|---|
| Erfindungsgem. Zusammensetzung A, B, C, D, E oder F nach Beispiel 1 | 0,10 GT |
| Milchsäure 80%ig | 0,50 GT |
| Parfüm | 0,08 GT |
| Wasser | 87,32 GT |

Beispiel 9

| Desodorierendes Mittel (flüssig) gegen Haargeruch | |
|---|---|
| Polyethylenglykol 400 | 0,20 GT |
| Ethanol | 37,50 GT |
| Parfüm | 0,10 GT |
| Erfindungsgem. Zusammensetzung A, B, C, D, E oder F nach Beispiel 1 | 0,10 GT |
| Hydriertes Rizinusöl 40 EO (Cremophor$^R$RH 40) | 0,20 GT |
| Citronensäure | 0,01 GT |
| Wasser | 61,89 GT |

Beispiel 10

| Desodorierende Seife | |
|---|---|
| Grundseife 80/20 (ca. 78% Fettsäure) | 96,84 GT |
| Überfettungsmittel | 1,45 GT |
| Farbstoffe | 0,01 GT |
| Antioxydans | 0,05 GT |
| Parfum | 1,07 GT |
| Titandioxid | 0,19 GT |
| Erfindungsgem. Zusammensetzung A, B, C, D, E oder F nach Beispiel 1 | 0,39 GT |
| | 100,00 GT |

**Patentansprüche**

1. Desodorierende oder antibakterielle Zusammensetzungen, insbesondere zur Verwendung in kosmetischen oder topischen Zubereitungen, dadurch gekennzeichnet, daß sie

    a) mindestens ein 3,7,11-Trimethyl-2,6,10-dodecatrien-1-ol,
    b) mindestens einen Phenylhydroxyalkylether mit einem, zwei oder drei Kohlenstoffatomen im Alkylrest,
    c) mindestens einen Glycerinmonoester einer Fettsäure mit 3-11 Kohlenstoffatomen und
    d) gegebenenfalls Glycerylmonolaurat enthalten.

2. Zusammensetzungen gemäß Anspruch 1, dadurch gekennzeichnet, daß sie 1 - 20 Gew.-% Glycerinmonolaurat enthalten.

3. Zusammensetzungen mit den Komponenten a), b) und c) gemäß Anspruch 1, dadurch gekennzeichnet, daß sie, jeweils bezogen auf das Gesamtgewicht der Zusammensetzungen,

    a) 10 - 50, vorzugsweise 15 - 40 Gew.-% eines oder mehrerer 3,7,11-Trimethyl-2,6,10-dodecatrien-1-ole,

b) 5 - 45, vorzugsweise 10 - 35 Gew.-%, mindestens eines Phenylhydroxyalkylethers mit maximal 3 C-Atomen im Alkylrest,

c) 10 - 80, vorzugsweise 25 - 75 Gew.-% mindestens eines Glycerinmonoesters einer Fettsäure mit 3-11 Kohlenstoffatomen

enthalten.

4. Zusammensetzungen mit den Komponenten a), b), c) und d) gemäß Anspruch 1, dadurch gekennzeichnet, daß sie, jeweils bezogen auf das Gesamtgewicht der Zusammensetzungen,

a) 10 - 45, vorzugsweise 15 - 35 Gew.-% eines oder mehrerer 3,6,11-Trimethyl-2,6,10-dodecatrien-1-ole,

b) 5 - 40, vorzugsweise 10 - 30 Gew.-%, mindestens eines Phenylhydroxyalkylethers mit maximal 3 C-Atomen im Alkylrest,

c) 10 - 70, vorzugsweise 25 - 65 Gew.-% mindestens eines Glycerinmonoesters einer Fettsäure mit 3-11 Kohlenstoffatomen,

d) 1 - 20, vorzugsweise 5 - 15 Gew.-% Glycerinmonolaurat

enthalten.

5. Desodorierende kosmetische Mittel,enthaltend neben üblichen Bestandteilen als desodorierenden Wirkstoff eine wirksame Menge der Zusammensetzungen nach Anspruch 1.

6. Desodorierende kosmetische Mittel nach Anspruch 5, gekennzeichnet durch einen Gehalt von 0,01 bis 10 Gew.-%, bezogen auf die Gesamtmenge des kosmetischen Mittels, der Zusammensetzungen nach Anspruch 1.

7. Verwendung der Zusammensetzungen nach Anspruch 1 als antibakterielle Wirkstoffe zur Stabilisierung topischer oder kosmetischer Zubereitungen.

8. Verwendung der Zusammensetzung nach Anspruch 1 als antibakterieller Wirkstoff zur Stabilisierung topischer oder kosmetischer Zubereitungen in einer Menge von 0,1 bis 10 Gew.% bezogen auf die Zubereitung.

9. Zusammensetzung nach Anspruch 1, dadurch gekennzeichnet, daß der Phenylhydroxyalkylether mit maximal 3 C-Atomen im Alkylrest 2-Phenoxyethanol ist.

10. Zusammensetzung nach Anspruch 1, dadurch gekennzeichnet, daß der Glycerylmonoester Glycerylmonocaprylat ist.

**Claims**

1. Deodorizing or antibacterial compositions, in particular for use in cosmetic or topical formulations, characterized in that they comprise

a) at least one 3,7,11-trimethyl-2,6,10-dodecatrien-1-ol,
b) at least one phenyl hydroxyalkyl ether having one, two or three carbon atoms in the alkyl radical,
c) at least one glycerol monoester of a fatty acid having 3 - 11 carbon atoms and
d) optionally glycerol monolaurate.

2. Compositions according to Claim 1, characterized in that they comprise 1 - 20% by weight of glycerol monolaurate.

3. Compositions with components a), b) and c) according to Claim 1, characterized in that they comprise, in each case based on the total weight of the compositions,

a) 10 - 50, preferably 15 - 40% by weight of one or more 3,7,11-trimethyl-2,6,10-dodecatrien-1-ols,

b) 5 - 45, preferably 10 - 35% by weight of at least one phenyl hydroxyalkyl ether having a maximum of 3 C atoms in the alkyl radical and

c) 10 - 80, preferably 25 - 75% by weight of at least one glycerol monoester of a fatty acid having 3 - 11 carbon atoms.

4. Compositions with components a), b), c) and d) according to Claim 1, characterized in that they comprise, in each case based on the total weight of the compositions,

a) 10 - 45, preferably 15 - 35% by weight of one or more 3,7,11-trimethyl-2,6,10-dodecatrien-1-ols,

b) 5 - 40, preferably 10 - 30% by weight of at least one phenyl hydroxyalkyl ether having a maximum of 3 C atoms in the alkyl radical,

c) 10 - 70, preferably 25 - 65% by weight of at least one glycerol monoester of a fatty acid having 3 - 11 carbon atoms and

d) 1 - 20, preferably 5 - 15% by weight of glycerol monolaurate.

5. Deodorizing cosmetic compositions comprising, in addition to customary constituents, an active amount of the compositions according to Claim 1 as the deodorizing active compound.

6. Deodorizing cosmetic compositions according to Claim 5, characterized in that they contain 0.01 to 10% by weight, based on the total amount of the cosmetic composition, of the compositions according to Claim 1.

7. Use of the compositions according to Claim 1 as antibacterial active compounds for stabilizing topical or cosmetic formulations.

8. Use of the compositions according to Claim 1 as an antibacterial active compound for stabilizing topical or cosmetic formulations in an amount of 0.1 to 10% by weight, based on the formulation.

9. Compositions according to Claim 1, characterized in that the phenyl hydroxyalkyl ether having a maximum of 3 C atoms in the alkyl radical is 2-phenoxyethanol.

10. Compositions according to Claim 1, characterized in that the glycerol monoester is glycerol monocaprylate.

**Revendications**

1. Compositions déodorantes ou antibactériennes, en particulier pour une utilisation dans des préparations cosmétiques ou topiques, caractérisées en ce qu'elles comprennent

a) au moins un 3,7,11-triméthyl-2,6,10-dodécatriène-1-ol,

b) au moins un phénylhydroxyalkyléther ayant un, deux ou trois atomes de carbone dans le radical alkyle,

c) au moins un monoester de glycérol et d'un acide gras ayant de 3 à 11 atomes de carbone, et

d) éventuellement du monolaurate de glycéryle.

2. Compositions selon la revendication 1, caractérisées en ce qu'elles comprennent de 1 à 20% en poids de monolaurate de glycérol.

3. Compositions comprenant les composants a), b) et c) selon la revendication 1, caractérisées en ce qu'elles comprennent, dans chaque cas par rapport au poids total des compositions

a) de 10 à 50, de préférence de 15 à 40% en poids d'un ou plusieurs 3,7,11-triméthyl-2,6,10-dodécatrién-1-ols,

b) de 5 à 45, de préférence de 10 à 35% en poids d'au moins un phénylhydroxyalkyléther ayant au plus 3 atomes de carbone dans le radical alkyle,

c) de 10 à 80, de préférence de 25 à 75% en poids d'au moins un monoester de glycérol et d'un acide gras ayant de 3 à 11 atomes de carbone.

4. Compositions comprenant les composants a), b), c) et d) selon la revendication 1, caractérisées en ce qu'elles comprennent, dans chaque cas par rapport au poids total des compositions

a) de 10 à 45, de préférence de 15 à 35% en poids d'un ou plusieurs 3,6,1 -triméthyl-2,6,10-dodécatrién-1-ols,
b) de 5 à 40, de préférence de 10 à 30% en poids d'au moins un phénylhydroxyalkyléther ayant au plus 3 atomes de carbone dans le radical alkyle,
c) de 10 à 70, de préférence de 25 à 65% en poids d'au moins un monoester de glycérol et d'un acide gras ayant de 3 à 11 atomes de carbone,
d) de 1 à 20, de préférence de 5 à 15% en poids de monolaurate de glycérol.

5.  Produits cosmétiques déodorants comprenant en tant que substance active déodorante, une quantité active des compositions selon la revendication 1, en plus des constituants habituels.

6.  Produits cosmétiques déodorants selon la revendication 5, caractérisés par une teneur de 0,01 à 10% en poids, par rapport à la quantité totale du produit cosmétique, en compositions selon la revendication 1.

7.  Utilisation des compositions selon la revendication 1 en tant que substances actives antibactériennes pour la stabilisation de préparations topiques ou cosmétiques.

8.  Utilisation des compositions selon la revendication 1, en tant que substance active antibactérienne pour la stabilisation de préparations topiques ou cosmétiques, en une quantité de 0,1 à 10% en poids, par rapport à la préparation.

9.  Composition selon la revendication 1, caractérisée en ce que le phénylhydroxyalkyléther ayant au plus 3 atomes de carbone dans le radical alkyle est le 2-phénoxyéthanol.

10. Composition selon la revendication 1, caractérisée en ce que le monoester glycérylique est le monocaprylate de glycéryle.